# EUROPEAN PATENT APPLICATION

(11) **EP 4 850 676 A1**
(43) Date of publication of application: **09.12.2026**
(21) Application number: 24922258.9
(22) Date of filing: 01.02.2024
(51) Int. Cl.: A61K 35/742, A23L 33/135, A61K 45/00, A61P 35/00, A61P 43/00

(54) **COMPOSITION FOR PROLIFERATING AND/OR ACTIVATING CD4-CD8-T CELLS**

(71) Applicant: Miyarisan Pharmaceutical Co., Ltd., Sakaki-machi Hanishina-gun Nagano 389-0682 (JP); National University Corporation Kumamoto University, Kumamoto 860-8555 (JP)
(72) Inventor: TOMITA, Yusuke, Kumamoto-shi, Kumamoto 860-8555 (JP); MOTOZONO, Chihiro, Kumamoto-shi, Kumamoto 860-8555 (JP); SAKAGAMI, Takuro, Kumamoto-shi, Kumamoto 860-8555 (JP); TAKAHASHI, Motomichi, Tokyo 114-0016 (JP); OKA, Kentaro, Tokyo 114-0016 (JP); HAYASHI, Atsushi, Tokyo 114-0016 (JP)
(74) Representative: J A Kemp LLP
(86) International application number: PCT/JP2024/003277
(87) International publication number: WO 2025/163852

(57) **Abstract**

Provided is a novel composition for proliferating and/or activating CD4⁻CD8⁻ T cells. The present invention relates to a composition for proliferating and/or activating CD4⁻CD8⁻ T cells, the composition containing, as an active ingredient, spore-forming bacteria or a cultured material thereof.

## Description

### TECHNICAL FIELD

The present invention relates to a composition for proliferating and/or activating CD4⁻CD8⁻ T cells.

### BACKGROUND ART

T cells are one kind of lymphocyte, and express a T cell receptor on the cell surface. Most of the T cells express either CD4 or CD8. On the other hand, there are CD4⁻CD8⁻ (double negative) T cells that do not express both CD4 and CD8.

CD4⁻CD8⁻ T cells have been reported to be associated with antitumor effects. Non-Patent Literature 1 reports that CD4⁻CD8⁻ T cells play an important role in immune homeostasis in a healthy state, and that they act as Treg cells, cytotoxic T cells, or Th cells and affect the innate and adaptive immune systems in distinct pathologies closely related to inflammatory diseases, autoimmune diseases, tumorigenesis, and oncogenesis. In addition, Non-Patent Literature 2 reports that CD4⁻CD8⁻ T cells may be a potential target of cancer immunotherapy.

### Citation List

### Non-Patent Literatures

Patent Literature 1: Wu Z, Zheng Y, Sheng J, Han Y, Yang Y, Pan H and Yao J (2022) CD3+CD4-CD8- (Double-Negative) T Cells in Inflammation, Immune Disorders and Cancer. Front. Immunol. 13:816005. doi: 10.3389/fimmu.2022.816005

Patent Literature 2: Okamura K, Nagayama S, Tate T, Kiyotani K, and Yusuke Nakamura (2020) The potential target of double negative T cells in cancer immunotherapy, Journal of Clinical Oncology 38:15_suppl, e15180

### SUMMARY OF INVENTION

An object of the present invention is to provide a novel composition for proliferating and/or activating CD4⁻CD8⁻ T cells.

The present inventors have surprisingly found that spore-forming bacteria can proliferate and/or activate CD4⁻CD8⁻ T cells. Based on this finding, the present inventors have completed the present invention.

That is, according to an aspect of the present invention, there is provided a composition for proliferating and/or activating CD4⁻CD8⁻ T cells, the composition containing, as an active ingredient, spore-forming bacteria or a cultured material thereof.

### BRIEF DESCRIPTION OF DRAWINGS

Fig. 1 shows an outline of an experiment of Test Example 1 and results thereof. A: a schematic diagram of the experiment, B: a scatter diagram showing results of flow cytometry in the case of using MIYA BM (registered trademark) suspension, and C: a graph showing results of flow cytometry in Test Example 1.
Fig. 2 shows experimental results of Test Example 2. A: a scatter diagram showing results of flow cytometry in the case of using a suspension of spores without a heat treatment, and B: a graph showing results of flow cytometry.
Fig. 3 shows experimental results of Test Example 3. A: a scatter diagram showing results of flow cytometry, and B: a graph showing results of flow cytometry.
Fig. 4 shows an outline of an experiment of Test Example 4 and results thereof. A: a schematic diagram of the experiment, B: a scatter diagram showing results of flow cytometry, and C: a graph showing results of flow cytometry.
Fig. 5 shows an outline of an experiment of Test Example 5 and results thereof. A: a schematic diagram of the experiment, and B: a scatter diagram showing results of flow cytometry.

### DESCRIPTION OF EMBODIMENTS

Hereinafter, embodiments according to an aspect of the present invention will be described. The present invention is not limited only to the following embodiments.

In the present specification, the phrase "X to Y" indicating a range means "X or more and Y or less". Unless otherwise specified, operations and measurements of physical properties and the like are measured under the conditions of room temperature (20 to 25°C)/relative humidity of 40 to 50% RH. In the present specification, "A and/or B" means at least one of A and B, and includes both A and B or either A or B.

### <Composition for proliferating and/or activating CD4⁻CD8⁻ T cells>

An aspect of the present invention is a composition for proliferating and/or activating CD4⁻CD8⁻ T cells, the composition containing, as an active ingredient, spore-forming bacteria or a cultured material thereof. According to an aspect of the present invention, there is provided a novel composition for proliferating and/or activating CD4⁻CD8⁻ T cells.

In the present specification, the composition for proliferating and/or activating CD4⁻CD8⁻ T cells is also simply referred to as the "composition according to the present invention".

Spore-forming bacteria are bacteria that form spores exhibiting very strong durability against a physical treatment, a chemical treatment, and the like. Formation of spores can be confirmed by microscopic observation, staining properties by a spore staining method or the like, heat resistance due to survivability after heating, resistance to an organic solvent or a disinfectant due to survivability after exposure, and the like.

In the composition according to the present invention, the type of the spore-forming bacteria is not particularly limited. Examples of the spore-forming bacteria include bacteria of the genus Clostridium, the genus Bacillus, the genus Amphibacillus, the genus Sporosarcina, and the like.

In one embodiment, the spore-forming bacteria are at least one kind of bacteria selected from the group consisting of bacteria of the genus Clostridium and the genus Bacillus.

Examples of the bacteria of the genus Clostridium include Clostridium butyricum, Clostridium absonum, Clostridium acetobutylicum, Clostridium beijerinckii, Clostridium cadaveris, Clostridium clostridiiforme, Clostridium cochlearium, Clostridium difficile, Clostridium innocuum, Clostridium novyi, Clostridium perfringens, Clostridium putrificum, Clostridium ramosum, Clostridium rectum, Clostridium scatologenes, Clostridium (Paeniclostridium) sordellii, Clostridium sporogenes, Clostridium tertium, Clostridium tyrobutyricum, Clostridium aerotolerans, Clostridium aminophilum, Clostridium aminovalericum, Clostridium celerecrescens, Clostridium cellulosi, Clostridium coccoides, Clostridium hiranonis, Clostridium leptum, Clostridium nexile, Clostridium oroticum, Clostridium polysaccharolyticum, Clostridium populeti, Clostridium scindens, Clostridium sphenoides, Clostridium sporosphaeroides, Clostridium symbiosum, Clostridium xylanolyticum, Clostridium hylemonae, and the like.

Examples of the bacteria of the genus Bacillus include Bacillus cereus, Bacillus circulans, Bacillus clausii, Bacillus coagulans, Bacillus licheniformis, Bacillus polyfermenticus, Bacillus pumilus, Bacillus subtilis, and the like.

These bacteria can be obtained, for example, from National Institute of Technology and Evaluation (NITE), RIKEN Japan Collection of Microorganisms (JCM), National BioResource Project (NBRP), American Type Culture Collection (ATCC), German Collection of Microorganisms and Cell Cultures (DSMZ), and the like.

The spore-forming bacteria are preferably bacteria of the genus Clostridium, more preferably Clostridium butyricum, Clostridium sordellii, or Clostridium putrificum, and still more preferably Clostridium butyricum MIYAIRI 588 (FERM BP-2789), Clostridium sordellii JCM3814 strains, or Clostridium putrificum ATCC25784 strains. Clostridium butyricum MIYAIRI 588 (FERM BP-2789) was deposited as FERM BP-2789 with Fermentation Research Institute, Agency of Industrial Science and Technology (currently called National Institute of Technology and Evaluation, International Patent Organism Depositary) (Room 120, 2-5-8 Kazusakamatari, Kisarazu, Chiba 292-0818, Japan) on May 1, 1981, and was transferred to the International Depositary Authority under the Budapest Treaty on March 6, 1990 and was deposited as accession number FERM BP-2789.

The spore-forming bacteria may be used singly or in combination of two or more kinds thereof.

The spore-forming bacteria may be in the form of spores or may be in the form of vegetative cells. The spore-forming bacteria are preferably in the form of spores from the viewpoint that the effects of the present invention can be further exhibited. The spore-forming bacteria contained in the composition according to the present invention may be only spores, may be only vegetative cells, or may be both spores and vegetative cells.

A method for forming spores is not particularly limited, and a conventionally known method can be adopted depending on the spore-forming bacteria to be used.

In the present invention, the cultured material of the spore-forming bacteria means a culture in which the spore-forming bacteria are cultured, a supernatant obtained by centrifuging the culture, a residue containing the spore-forming bacteria obtained by centrifuging the culture, a purified product from the residue, and a dried product of the residue.

The purified product from the residue can be obtained according to a conventionally known method depending on the component of interest.

The dried product of the residue can be obtained by subjecting a residue containing bacteria obtained by centrifuging the culture to an air drying treatment or a reduced-pressure drying treatment.

The cultured material of the spore-forming bacteria according to the present invention can be obtained by a conventionally known culture method depending on the spore-forming bacteria to be used. For example, a culture medium to be used for culturing the spore-forming bacteria may be either a solid or liquid culture medium, and may be either a synthetic culture medium or a natural culture medium as long as it is a culture medium containing a carbon source that can be assimilated by the spore-forming bacteria to be used, an appropriate amount of a nitrogen source, inorganic salts, and other nutrients. Usually, the culture medium contains a carbon source, a nitrogen source, and an inorganic substance.

The carbon source that can be used in the culturing of the spore-forming bacteria according to the present invention is not particularly limited as long as the used strain is a carbon source that can be assimilated. Specific examples thereof include organic acids, such as gluconic acid, capric acid, adipic acid, malic acid, citric acid, phenyl acetate, acetic acid, lactic acid, succinic acid, glucuronic acid, and pyruvic acid, and salts thereof; hydrocarbons such as hexadecane; natural products such as wheat and rice; alcohols such as glycerol, methanol, and ethanol; saccharides; and the like in consideration of the assimilability of the spore-forming bacteria. The carbon source is appropriately selected in consideration of the assimilability by the spore-forming bacteria to be cultured. One kind or two or more kinds of the carbon sources can be selectively used.

Examples of the nitrogen source that can be used in the culturing of the spore-forming bacteria according to the present invention include organic nitrogen sources such as a meat extract, a fish meat extract, peptone, polypeptone, tryptone, a yeast extract, a malt extract, a soybean hydrolysate, a soybean powder, casein, milk casein, casamino acid, various amino acids including glycine, glutamic acid, aspartic acid, and the like, corn steep liquor, and hydrolysates of other animals, plants, and microorganisms; inorganic nitrogen sources such as ammonia, ammonium salts including ammonium nitrate, ammonium sulfate, ammonium chloride, and the like, nitrates including sodium nitrate, nitrites including sodium nitrite and the like, and urea; and the like. The nitrogen source is appropriately selected in consideration of the assimilability by the spore-forming bacteria to be cultured. One kind or two or more kinds of the nitrogen sources can be selectively used.

Examples of the inorganic substance that can be used in the culturing of the spore-forming bacteria according to the present invention include phosphates, hydrochlorides, sulfates, acetates, carbonates, and halides such as chlorides, of magnesium, manganese, calcium, sodium, potassium, copper, iron, zinc, and the like. The inorganic substance is appropriately selected in consideration of the assimilability by the spore-forming bacteria to be cultured. One kind or two or more kinds of the inorganic substances can be selectively used. A surfactant or the like may be added to the culture medium as necessary.

The spore-forming bacteria according to the present invention can be cultured by an ordinary method. For example, depending on the type of spore-forming bacteria, the spore-forming bacteria are cultured under aerobic or anaerobic conditions. In the former case, the spore-forming bacteria are cultured by shaking, aeration stirring, or the like. The spore-forming bacteria may be cultured continuously or in batches. The culture conditions are appropriately selected depending on the composition of a culture medium and a culture method, and are not particularly limited as long as the spore-forming bacteria according to the present invention can grow, and can be appropriately selected according to the type of spore-forming bacteria to be cultured. The culture temperature is usually 20 to 42°C and preferably 35 to 40°C. The pH of a culture medium suitable for culturing is not particularly limited, but is preferably 5 to 11 and more preferably 6 to 10. The culture time is not particularly limited, and varies depending on the type of spore-forming bacteria to be cultured, the amount of a culture medium, culture conditions, and the like. Usually, the culturing is performed up to the exponential growth phase in the case of vegetative cells, and is performed, for example, for 16 hours to 7 days in the case of spores.

As an example of the cultured material of the spore-forming bacteria according to the present invention, a method for preparing a cultured material of Clostridium butyricum MIYAIRI 588 (FERM BP-2789) (hereinafter, also referred to as 588 strains) will be described.

As a method for preparing a culture of vegetative cells, the method described in JP H08-252088 A can be used.

Specifically, 588 strains are inoculated into a culture medium (PYD culture medium) including 1.0% peptone, 1.5% yeast extract, and 3% glucose at 10⁶/mL, and the mixture is gently stirred at 27°C and 20 rpm. Culturing is performed until the early stage of the exponential growth phase while controlling the set pH range to 5.5 ± 0.2 with a 5% aqueous sodium hydroxide solution using a pH controller. Thereby, a "culture of vegetative cells of 588 strains" can be obtained.

As a method for preparing a suspension of spores, the method described in R. SATO and M. TANAKA, Multiplication of Orally Administered Clostridium Butyricum in Rats, MICROBIAL ECOLOGY IN HEALTH AND DISEASE, Vol. 9: 115-122 (1996) can be used.

Specifically, a "suspension of spores of 588 strains" can be obtained by culturing at 37°C for 5 days under anaerobic conditions using a normal agar culture medium added with 0.5% glucose and 0.3% calcium carbonate, and then suspending in a liquid such as a culture medium. A "purified spore suspension containing no vegetative cells of 588 strains" can also be obtained by suspending in sterilized water instead of a culture medium, then centrifugally washing with sterilized water, purifying by a density gradient centrifugation method using 45% urografin (common name: amidotrizoic acid), and further centrifugally washing with sterilized water.

As the method for preparing a suspension of spores, the method described in JP H11-042081 A may be used.

Specifically, 588 strains are inoculated into 10 ml of a CS liquid culture medium including 2.0% corn starch, a 2.0% amino acid solution, and 0.75% calcium carbonate, and anaerobically cultured at 35 to 37°C for 16 to 24 hours by a carbon dioxide gas replacement steel wool method to prepare a preculture. After completion of the culturing for a predetermined time, 0.01 to 1 ml of this preculture is inoculated into 10 ml of a CS liquid culture medium for main culturing at 60 to 100°C, and then the temperature of the CS liquid culture medium is cooled to 30 to 40°C with water, and anaerobically cultured at 35 to 37°C for 40 to 48 hours by a carbon dioxide gas replacement steel wool method. Thereby, a "suspension of spores of 588 strains" can be obtained.

The obtained culture is centrifuged (2,000 to 6,000 g × 10 to 30 minutes) to separate a "residue containing bacteria obtained by centrifuging the culture", and the residue is subjected to a drying treatment by air drying or the like at 0 to 80°C, preferably 10 to 20°C, for 1 to 24 hours, preferably 5 to 18 hours, or a drying treatment under reduced pressure at 0 to 80°C, preferably 10 to 20°C, 0.05 to 500 Torr (7 Pa to 66.7 kPa), preferably 1 to 100 Torr (133 Pa to 13.3 kPa), for 1 to 24 hours, preferably 2 to 15 hours, thereby obtaining a "dried product of the residue". In order to obtain a dried product, spray drying, freeze drying, or the like may be used.

The spore-forming bacteria or a cultured material thereof according to the present invention can proliferate and/or activate CD4⁻CD8⁻ T cells.

In the present specification, proliferation of CD4⁻CD8⁻ T cells can be confirmed by CTV (Celltrace violet) assay. Activation of CD4⁻CD8⁻ T cells can be confirmed by AIM (Activation-InducedMarker) assay.

In the present specification, CD4⁻CD8⁻ T cells mean CD4-negative CD8-negative T cells. In a preferred embodiment, from the viewpoint that the effects of the present invention can be further exhibited, the CD4⁻CD8⁻ T cells include γδ T cells, and the γδ T cells include Vγ9Vδ2 T cells.

The composition according to the present invention contains the spore-forming bacteria or a cultured material thereof according to the present invention in an amount sufficient to exhibit a desired effect (that is, an effective amount). The composition according to the present invention may be the spore-forming bacteria or a cultured material thereof itself according to the present invention, or may further contain other components. Examples of the other components include the components described in the forms of a pharmaceutical composition and a food and beverage composition described later.

In one embodiment, the composition according to the present invention is used in combination with an antitumor agent.

In the present specification, the term "in combination with" means simultaneous administration of the composition according to the present invention and the antitumor agent, and sequential administration of the composition according to the present invention and the antitumor agent at predetermined intervals, respectively, over a treatment period (combination therapy). The administration route and administration means of the composition according to the present invention and the antitumor agent may be the same or different.

As described above, the CD4⁻CD8⁻ T cells have been reported to be associated with antitumor effects. When the composition according to the present invention is used in combination with an antitumor agent, the effect of the antitumor agent can be expected to be further increased.

The antitumor agent is not particularly limited, and examples thereof include a tyrosine kinase inhibitor, an immune checkpoint inhibitor, an alkylating agent, an antimetabolite, an antitumor antibiotic, a plant-derived antitumor agent, an antitumor platinum coordination compound, an antitumor camptothecin derivative, a serine threonine kinase inhibitor, a kinase inhibitor, an antitumor monoclonal antibody, an interferon preparation, a biological response modifier, a hormone preparation, an angiogenesis inhibitor, an epigenetic related molecule inhibitor, a post-translational protein modification inhibitor, a proteasome inhibitor, and the like. The antitumor agent is preferably a tyrosine kinase inhibitor or an immune checkpoint inhibitor.

The tyrosine kinase inhibitor is a drug that exhibits an antitumor effect by inhibiting tyrosine kinase of cells, which is an enzyme involved in many cellular functions such as cell signaling, growth, and division.

The tyrosine kinase inhibitor is not particularly limited, and a conventionally known tyrosine kinase inhibitor can be used. The tyrosine kinase inhibitor is preferably at least one selected from the group consisting of an EGFR tyrosine kinase inhibitor, a c-kit receptor tyrosine kinase inhibitor, a BCR-ABL tyrosine kinase inhibitor, and a multikinase inhibitor, and more preferably a multikinase inhibitor.

Specific examples of the tyrosine kinase inhibitor include imatinib, dasatinib, nilotinib, afatinib, erlotinib, osimertinib, gefitinib, axitinib, cabozantinib, sunitinib, sorafenib, pazopanib, regorafenib, lenvatinib, and the like.

The immune checkpoint inhibitor inhibits the function of an immune checkpoint of a receptor or a ligand, and examples thereof include an antagonist for a suppressive receptor and an agonist for a co-stimulatory immune checkpoint receptor.

The term "antagonist" includes various substances that interfere with receptor activation induced by binding between a receptor and a ligand. Examples thereof include substances that interfere with the binding between a receptor and a ligand by binding to the receptor, and substances that interfere with the binding between a receptor and a ligand by binding to the ligand.

Examples of an antagonist against an inhibitory immune checkpoint include an antagonistic antibody that binds to an inhibitory immune checkpoint molecule (inhibitory receptor or a ligand of the receptor), a soluble polypeptide that is designed based on an inhibitory immune checkpoint ligand and does not activate the receptor, a vector capable of expressing such a polypeptide, and the like.

The immune checkpoint inhibitor is not particularly limited, and a conventionally known immune checkpoint inhibitor can be used. The immune checkpoint inhibitor is preferably at least one selected from the group consisting of a PD-1 inhibitor, a PD-L1 inhibitor, and a CTLA-4 inhibitor, and more preferably a PD-1 inhibitor.

Specific examples of the immune checkpoint inhibitor are not particularly limited, but are at least one selected from the group consisting of nivolumab, ipilimumab, pembrolizumab, semipilimumab, durvalumab, daclizumab, avelumab, and atezolizumab, and more preferably at least one selected from the group consisting of nivolumab, pembrolizumab, and semipilimumab.

The dosage and administration method of the composition according to the present invention and the antitumor agent can be appropriately determined in consideration of the type of tumor, the symptom, age, sex, body weight, and condition of the subject, and the like.

In one embodiment, the composition according to the present invention is a pharmaceutical composition. As described above, the CD4⁻CD8⁻ T cells have been reported to be associated with antitumor effects, and can be used in medical use.

The pharmaceutical composition according to the present invention contains the spore-forming bacteria or a cultured material thereof according to the present invention in an amount sufficient to exhibit a desired effect (that is, an effective amount).

The pharmaceutical composition according to the present invention may be the spore-forming bacteria or a cultured material thereof itself according to the present invention, or may be prepared as an oral preparation or a parenteral preparation in combination with a pharmaceutically acceptable additive according to a conventional method. Examples of the pharmaceutically acceptable additive include an excipient, a stabilizer, a preservative, a wetting agent, an emulsifier, a lubricant, a sweetener, a coloring agent, a fragrance, a buffering agent, an antioxidant, a pH adjusting agent, a binder, a thickener, a dispersant, a suspending agent, a disintegrant, a bacteriostat, a surfactant, and the like. The dosage form is not particularly limited, and may be appropriately set, and examples thereof include a tablet, a powder, a fine granule, a granule, a capsule, a pill, a sustained-release preparation, a solution, a suspension, an emulsion, a lotion, an injection solution, a drop solution, an external preparation, a suppository, a patch, and the like.

In one embodiment, the composition according to the present invention is a food and beverage composition.

The food and beverage may be a food and beverage prepared by adding a conventional additive component such as a stabilizer to the composition according to the present invention, a food and beverage prepared by further blending various proteins, saccharides, fats, trace elements, vitamins, and the like, a liquid, semi-liquid, or solid form, a paste form, or a food and beverage obtained by adding the composition according to the present invention to a general food and beverage.

In the present specification, the "food and beverage" is not particularly limited as long as it is a product other than a medicine and is in a form that can be orally ingested by a mammal or the like, and the form may be any of a liquid product (a solution, a suspension, an emulsion, or the like), a semi-liquid product, a powder, or a solid molded product. For this reason, the food and beverage may be in the form of, for example, a beverage, or may be in the form of a tablet of a nutritional supplement such as a supplement.

Specific examples of the food and beverage include convenience foods such as instant noodles, retort pouch foods, canned foods, microwave meals, instant soups/miso soups, and freeze-dried foods; beverages such as soft drinks, fruit juice drinks, vegetable drinks, soy milk drinks, coffee drinks, tea drinks, powdered drinks, concentrated drinks, and alcoholic drinks; wheat flour products such as bread, pasta, noodles, cakes mixes, seasoned fry mix powder, and bread crumbs; confectionery foods such as candies, caramels, chewing gum, chocolates, cookies, biscuits, cakes, pies, snacks, crackers, Japanese sweets, and dessert sweets; condiments such as sauces, processed tomato seasonings, flavorings, cooking mixes, sauce items, dressings, soups, and curry/stew sauce mixes; oils and fats such as processed oils, butter, margarine, and mayonnaise; dairy products such as milk drinks, yogurts, lactic acid bacteria beverages, ice cream, and cream; processed marine products such as fish meat ham/sausages and fish paste products; livestock processed products such as meat hams/sausages; processed agricultural products such as canned agricultural foods, jam/marmalade, pickled products, boiled beans, and cereals; frozen foods; nutritional foods; and the like.

In the present specification, the term "food and beverage" includes foods classified as health foods, functional foods, foods for specified health uses, dietary supplements, foods labeled with disease risk reduction, or foods for patients. When the term "food and beverage" is used for a mammal other than a human, it may be used in a sense including feed.

In the food and beverage composition according to the present invention, a component having another function may be further added. For example, by blending the active ingredient of the present invention in foods, health foods, functional foods, and supplements (for example, foods containing one or more of minerals such as calcium and magnesium, and vitamins such as vitamin K) taken in daily life, it is possible to provide foods and beverages having a function based on other ingredients in addition to the effect according to the present invention.

### <Method for proliferating and/or activating CD4⁻CD8⁻ T cells>

Another aspect of the present invention is a method for proliferating and/or activating CD4⁻CD8⁻ T cells, the method including bringing peripheral blood mononuclear cells into contact with spore-forming bacteria or a cultured material thereof. The spore-forming bacteria or a cultured material thereof according to the present invention can remarkably proliferate and/or activate CD4⁻CD8⁻ T cells in peripheral blood mononuclear cells.

The method for preparing and isolating peripheral blood mononuclear cells (PBMCs) is not particularly limited, and can be performed according to a conventionally known method. Peripheral blood mononuclear cells can be isolated by removing plasma components, red blood cells, platelets, granulocytes, and the like from a blood sample collected from a subject using, for example, a density gradient centrifugation method.

A subject from which the peripheral blood mononuclear cells are derived is not particularly limited as long as it is a mammal. The mammal includes both primates such as human, monkey, gorilla, chimpanzee, and orangutan, and non-human mammals such as mouse, rat, hamster, guinea pig, rabbit, dog, cat, pig, cow, horse, sheep, camel, and goat. Among these, the subject is preferably a primate and more preferably a human.

In the present aspect, the "spore-forming bacteria or a cultured material thereof" is the same as the matter described above, and thus the description thereof is omitted.

The method of bringing peripheral blood mononuclear cells into contact with spore-forming bacteria or a cultured material thereof is not particularly limited, and examples thereof include a method in which culturing is performed in a culture medium containing peripheral blood mononuclear cells and spore-forming bacteria or a cultured material thereof. When culturing is performed in a culture medium containing peripheral blood mononuclear cells and spore-forming bacteria or a cultured material thereof, culture conditions such as a culture temperature and a culture time can be adjusted to known culture conditions of peripheral blood mononuclear cells.

In the present aspect, T cells isolated from a blood sample or peripheral blood mononuclear cells instead of peripheral blood mononuclear cells can be used. The isolation of T cells can be performed by a conventionally known method.

One embodiment of the present aspect is a method for proliferating and/or activating CD4⁻CD8⁻ T cells, the method including bringing T cells into contact with spore-forming bacteria or a cultured material thereof.

Another aspect of the present invention is a method for proliferating and/or activating CD4⁻CD8⁻ T cells, the method comprising administering an effective amount of spore-forming bacteria or a cultured material thereof to a subject.

In the present aspect, the "spore-forming bacteria or a cultured material thereof" is the same as the matter described above, and thus the description thereof is omitted. Since the "subject" is the same as the "subject from which the peripheral blood mononuclear cells are derived" described above, the description thereof is omitted.

In the present aspect, the "effective amount" means an amount of an active ingredient (that is, spore-forming bacteria or a cultured material thereof) required for proliferation and/or activation of CD4⁻CD8⁻ T cells.

In the present aspect, the spore-forming bacteria and a cultured material thereof may be administered orally, intravenously, intramuscularly, intrathecally, intraperitoneally, or transdermally (for example, as an ointment), or by inhalation. The spore-forming bacteria or a cultured material thereof may be prepared as an oral preparation or a parenteral preparation in combination with a pharmaceutically acceptable additive according to a conventional method according to each of these administration forms. The pharmaceutically acceptable additive is the same as those described above, and thus the description thereof is omitted.

Embodiments of the present invention are illustrated below.
[1] A composition for proliferating and/or activating CD4⁻CD8⁻ T cells, the composition containing, as an active ingredient, spore-forming bacteria or a cultured material thereof.
[2] The composition according to [1], wherein the spore-forming bacteria are at least one kind of bacteria selected from a group consisting of bacteria of genus Clostridium and genus Bacillus.
[3] The composition according to [1] or [2], wherein the spore-forming bacteria are at least one kind of bacteria selected from a group consisting of Clostridium butyricum, Clostridium absonum, Clostridium acetobutylicum, Clostridium beijerinckii, Clostridium cadaveris, Clostridium clostridiiforme, Clostridium cochlearium, Clostridium difficile, Clostridium innocuum, Clostridium novyi, Clostridium perfringens, Clostridium putrificum, Clostridium ramosum, Clostridium rectum, Clostridium scatologenes, Clostridium sordellii, Clostridium sporogenes, Clostridium tertium, Clostridium tyrobutyricum, Clostridium aerotolerans, Clostridium aminophilum, Clostridium aminovalericum, Clostridium celerecrescens, Clostridium cellulosi, Clostridium coccoides, Clostridium hiranonis, Clostridium leptum, Clostridium nexile, Clostridium oroticum, Clostridium polysaccharolyticum, Clostridium populeti, Clostridium scindens, Clostridium sphenoides, Clostridium sporosphaeroides, Clostridium symbiosum, Clostridium xylanolyticum, Clostridium hylemonae, Bacillus cereus, Bacillus circulans, Bacillus clausii, Bacillus coagulans, Bacillus licheniformis, Bacillus polyfermenticus, Bacillus pumilus, and Bacillus subtilis.
[4] The composition according to [3], wherein the spore-forming bacteria are at least one kind of bacteria selected from a group consisting of Clostridium butyricum, Clostridium sordellii, and Clostridium putrificum.
[5] The composition according to any one of [1] to [4], wherein the spore-forming bacteria are in a form of spores.
[6] The composition according to any one of [1] to [5], wherein the CD4⁻CD8⁻ T cells include γδ T cells.
[7] The composition according to [6], wherein the γδ T cells include Vγ9Vδ2 T cells.
[8] The composition according to any one of [1] to [7], which is used in combination with an antitumor agent.
[9] The composition according to any one of [1] to [8], which is a pharmaceutical composition.
[10] The composition according to any one of [1] to [7], which is a food and beverage composition.
[11] A method for proliferating and/or activating CD4⁻CD8⁻ T cells, the method including bringing peripheral blood mononuclear cells into contact with spore-forming bacteria or a cultured material thereof.
[12] A method for proliferating and/or activating CD4⁻CD8⁻ T cells, the method including administering an effective amount of spore-forming bacteria or a cultured material thereof to a subject.
[13] Spore-forming bacteria or a cultured material thereof being used for proliferating and/or activating CD4⁻CD8⁻ T cells.

### Examples

Hereinafter, the present invention will be described in detail using Examples. The present invention is not limited to the following Examples.

The following experiments were all performed after approval from the Ethics Committee of Kumamoto University. As a human cell specimen, peripheral blood mononuclear cells (PBMCs) fractionated from blood collected with heparin from a healthy person were used. All the blood samples from humans were collected after making informed consent and confirming the intention of the blood donor. The handling of a patient specimen was analyzed by coding without personal information.

### <Test Example 1>

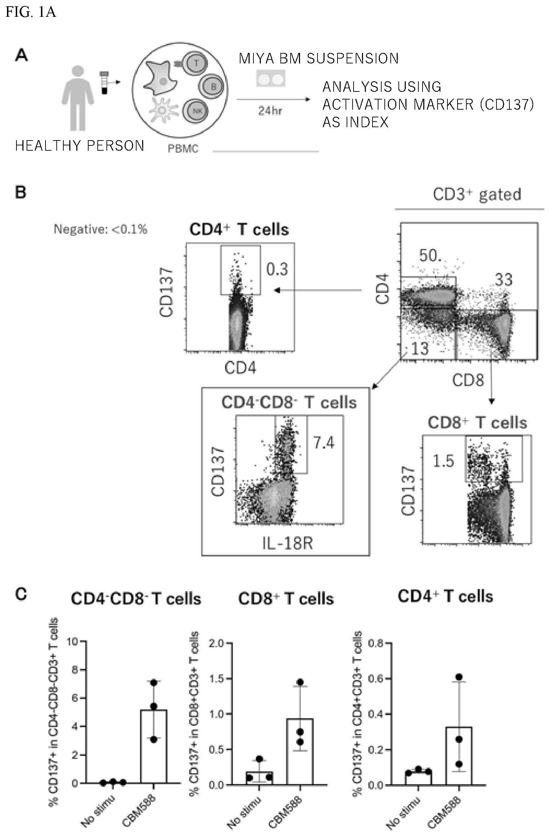

Using PBMCs derived from a healthy person, it was examined whether the activity of a specific T cell subset is induced by the stimulation of MIYA BM (registered trademark) tablet (manufactured by Miyarisan Pharmaceutical Co., Ltd.). The MIYA BM (registered trademark) tablet contains, as an active ingredient, Clostridium butyricum MIYAIRI 588 (FERM BP-2789) (588 strains). Specifically, AIM (Activation-Induced Marker) assay was performed, and analysis using an activation marker (CD137) as an index was performed by flow cytometry. 1×10⁶ PBMCs/well were suspended in 100 µL of 10% FBS-containing RPMI1640 culture medium (manufactured by Thermo Fisher Scientific Inc., Cat No: 200-02). One tablet of MIYA BM (registered trademark) tablet was dissolved in 3 mL of pure water and suspended. 3 µL, 10 µL, and 30 µL of MIYA BM (registered trademark) suspensions were each added to the wells. Non-stimulated wells of MIYA BM (registered trademark) tablet were placed as negative controls. After the pulse, culturing was performed for 24 hours under the conditions of 37°C and 2.5% CO₂. After the culturing, PBMCs were washed, and the surface was stained using an antibody. As antibodies, CD3 FITC (UCHT1, 100-fold dilution, manufactured by BioLegend, Inc.), CD8 APCcy7 (RPA-T8, 100-fold dilution, manufactured by BioLegend, Inc.), CD14 PerCP/Cy5.5 (HCD14, 100-fold dilution, manufactured by BioLegend, Inc.), CD19 PerCP/Cy5.5 (HIB19, 10-fold dilution, manufactured by BioLegend, Inc.), CD25 PEcy7 (M-A251, 50-fold dilution, manufactured by BioLegend, Inc.), and CD137 APC (4B4-1, 50-fold dilution, manufactured by BioLegend, Inc.) were used. For staining of dead cells, 7-aminoactinomycin D (7-AAD, manufactured by BioLegend, Inc., Cat No: 420404) was also added. After addition, the mixture was incubated on ice for 20 minutes. After incubation, PBMCs were immobilized using 1% paraformaldehyde (manufactured by NACALAI TESQUE, INC., Cat No: 09154-85). After immobilization, the expression level of surface molecules was measured by flow cytometry using FACS Canto II (manufactured by BD Biosciences). Data acquired by flow cytometry was analyzed using FACS Diva v9.0 (manufactured by BD Biosciences) and FlowJo software v10 (manufactured by Tree Star, Inc.). The results are shown in Figs. 1B and 1C. Fig. 1B shows the results in the case of using MIYA BM (registered trademark) suspension (10 µL). Fig. 1C shows the results in the case of using MIYA BM (registered trademark) suspension (10 µL) (CBM588) and results of the control (No stimu).

As shown in Figs. 1B and 1C, when stimulated with MIYA BM (registered trademark) tablet, significant activation was observed in CD4⁻CD8⁻ T cells, and a significant increase in activation markers of the CD4⁻CD8⁻ T cell subset was observed compared to no stimulation (Nostimu).

### <Test Example 2>

A culture or suspension of 588 strains as a constituent component of MIYA BM was prepared. As a culture or suspension of 588 strains, a suspension of spores without a heat treatment, a suspension of spores with a heat treatment, a culture of vegetative cells without a heat treatment, and a culture of vegetative cells with a heat treatment were prepared. The heat treatment was performed at 90°C for 90 minutes for spores and was performed at 70°C for 30 minutes for vegetative cells. It was examined whether or not the activation of the same subset as in Test Example 1 was obtained by adding the same PBMCs derived from a healthy person as in Test Example 1 and performing AIM assay. Spores of 588 strains were prepared by the following method. Culturing was performed at 37°C for 5 days under anaerobic conditions using a normal agar culture medium added with 0.5% glucose and 0.3% calcium carbonate, and then centrifugal washing with sterilized water was performed. Thereafter, the residue was purified by a density gradient centrifugation method using 45% urografin (common name: amidotrizoic acid). A suspension of spores of 588 strains was prepared by centrifugally washing with sterilized water. Also in the following Test Examples, spores of 588 strains were similarly formed.

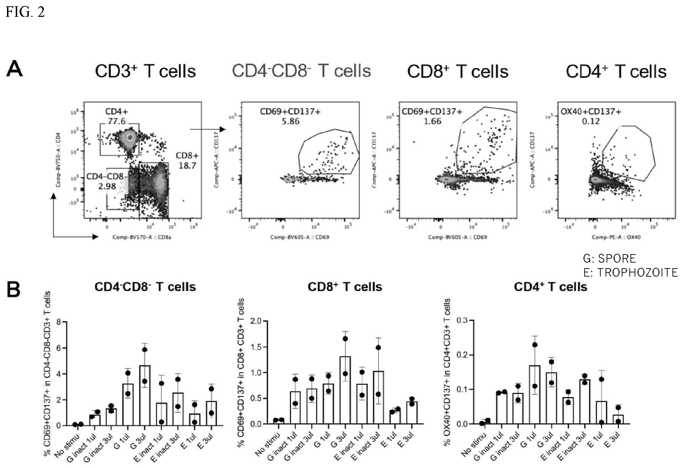

Specifically, AIM (Activation-Induced Marker) assay was performed, and analysis using an activation marker (CD69 OX40 CD137) as an index was performed by flow cytometry. 1×10⁶ PBMCs/well were suspended in 100 µL of 10% FBS-containing RPMI1640 culture medium (manufactured by Thermo Fisher Scientific Inc., Cat No: 200-02). 1 µL and 3 µL of culture of 588 strains were added to each well. The concentration of spores or vegetative cells in the culture was 1×10⁷ cells/mL. Wells without stimulation were placed as negative controls. After the pulse, culturing was performed for 24 hours under the conditions of 37°C and 2.5% CO₂. After the culturing, PBMCs were washed, and the surface was stained using the same antibody as in Test Example 1 and OX40 (Ber-ACT35, 50-fold dilution, manufactured by BioLegend, Inc.). For staining of dead cells, 7-aminoactinomycin D (7-AAD, manufactured by BioLegend, Inc., Cat No: 420404) was also added. After addition, the mixture was incubated on ice for 20 minutes. After incubation, PBMCs were immobilized using 1% paraformaldehyde (manufactured by NACALAI TESQUE, INC., Cat No: 09154-85). After immobilization, the expression level of surface molecules was measured by flow cytometry using FACS Canto II (manufactured by BD Biosciences). Data acquired by flow cytometry was analyzed using FACS Diva v9.0 (manufactured by BD Biosciences) and FlowJo software v10 (manufactured by Tree Star, Inc.). The results are shown in Figs. 2A and 2B. Fig. 2A shows the results when a suspension (3 µL) of spores without a heat treatment was used. In Fig. 2B, "No stimu" indicates a control, "G" indicates a suspension of spores without a heat treatment, "G inact" indicates a suspension of spores with a heat treatment, "E" indicates a culture of vegetative cells without a heat treatment, and "E inact" indicates a culture of vegetative cells with a heat treatment.

As shown in Fig. 2A, when stimulated with a suspension (3 µL) of spores without a heat treatment, significant activation is observed in CD4⁻CD8⁻ T cells. As shown in Fig. 2B, in the group stimulated with a culture of 588 strains, the activation of CD4⁻CD8⁻ T cells was significantly observed as compared with the group without stimulation, which was shown to be consistent with the response in the tablet of MIYA BM (registered trademark) tablet. When comparing the group stimulated with a culture of 588 strains, the activation of CD4⁻ CD8⁻ T cells was observed in the group stimulated with a suspension of spores as compared with the group stimulated with vegetative cells. Regarding the effect of the heat treatment on activation of CD4⁻CD8⁻ T cells, it can be seen that in the suspension of spores, the group without a heat treatment activated CD4⁻CD8⁻ T cells more than the group with a heat treatment. In the culture of vegetative cells, no effect on CD4⁻CD8⁻ T cells was observed between the group without a heat treatment and the group with a heat treatment.

### <Test Example 3>

Using PBMCs derived from a healthy person, identification of immune subset activated by spore stimulation of 588 strains was attempted using cell proliferation as an index. Specifically, cell proliferation was evaluated by performing CTV (Cell trace violet) assay, labeling PBMCs with a CTV dye, and detecting a decrease in concentration of the CTV dye according to cell division by flow cytometry.

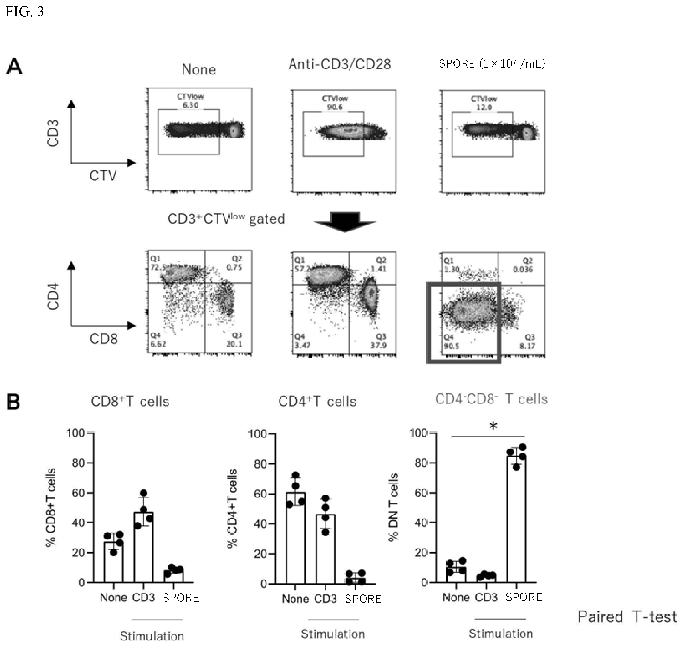

5 mM CTV/20 µL DMSO was diluted 5-fold with PBS by Cell Trace Violet Cell Proliferation Kit (Thermo Fisher Scientific, Invitrogen, C34557) to prepare a 1 mM CTV/µL solution. 10 mM CTV/10 µL was added dropwise to 1×10⁷ PBMCs/10 mL PBS and suspended, and labeled as a final concentration of 1 µM CTV/µL. 1×10⁶ CTV-labeled PBMCs/well were suspended in 200 µL of 10% FBS-containing RPMI1640 culture medium (manufactured by Thermo Fisher Scientific Inc., Cat No: 200-02). A suspension of spores without a heat treatment (spore concentration: 1×10⁷ cells/mL) was added thereto and suspended. Wells without stimulation and wells with stimulation with anti-CD3-CD28 antibody (2 µL) were placed as controls. After the pulse, culturing was performed for 7 days under the conditions of 37°C and 2.5% CO₂. After the culturing, PBMCs were washed, and the surface was stained using an antibody. As antibodies, CD3 FITC (UCHT1, 100-fold dilution, manufactured by BioLegend, Inc.), CD8 APCcy7 (RPA-T8, 100-fold dilution, manufactured by BioLegend, Inc.), CD14 PerCP/Cy5.5 (HCD14, 100-fold dilution, manufactured by BioLegend, Inc.), and CD19 PerCP/Cy5.5 (HIB19, 10-fold dilution, manufactured by BioLegend, Inc.) were used. For staining of dead cells, 7-aminoactinomycin D (7-AAD, manufactured by BioLegend, Inc., Cat No: 420404) was also added. After addition, the mixture was incubated on ice for 20 minutes. After incubation, PBMCs were immobilized using 1% paraformaldehyde (manufactured by NACALAI TESQUE, INC., Cat No: 09154-85). After immobilization, the expression level of surface molecules was measured by flow cytometry using Cytek Northern Lights (manufactured by Cytek Japan). Data acquired by flow cytometry was analyzed using FlowJo software v10 (manufactured by Tree Star, Inc.). The results are shown in Figs. 3A and 3B. In Fig. 3A, "None" indicates no stimulation, "Anti-CD3/CD28" indicates an anti-CD3-CD28 antibody, and "Spore" indicates a suspension of spores without a heat treatment. In Fig. 3B, "None" indicates no stimulation, "CD3" indicates an anti-CD3-CD28 antibody, and "Spore" indicates a suspension of spores without a heat treatment.

As shown in Figs. 3A and 3B, significant proliferation of CD4⁻CD8⁻ T cells was significantly observed in the group stimulated with a suspension of spores without a heat treatment compared to the group without stimulation and the group stimulated with an anti-CD3-CD28 antibody.

### <Test Example 4>

Using PBMCs derived from a healthy person, identification of subset constituting CD4⁻CD8⁻ T cells that remarkably proliferate under spore stimulation was attempted.

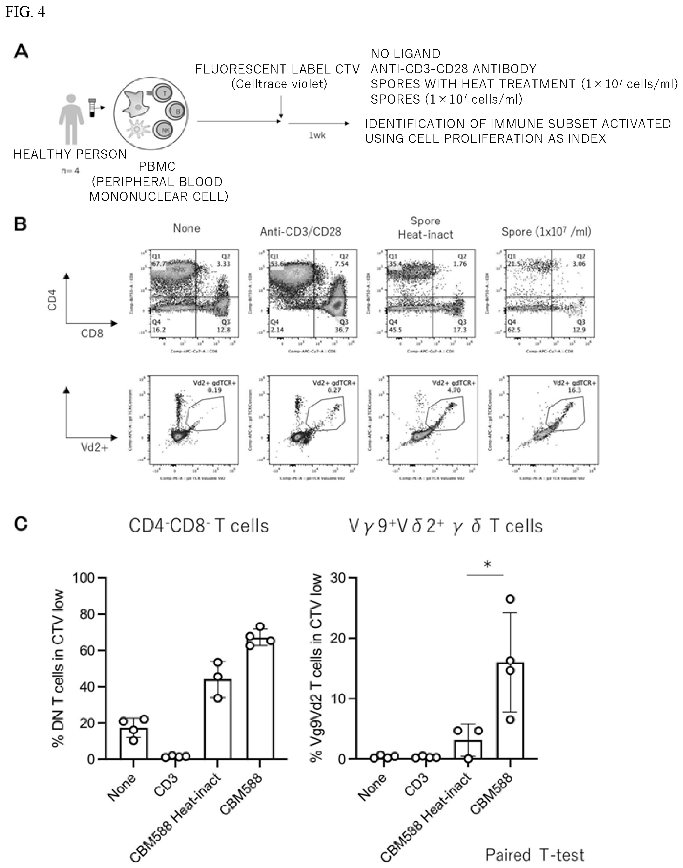

5 mM CTV/20 µL DMSO was diluted 5-fold with PBS by Cell Trace Violet Cell Proliferation Kit (Thermo Fisher Scientific, Invitrogen, C34557) to prepare a 1 mM CTV/µL solution. 10 mM CTV/10 µL was added dropwise to 1×10⁷ PBMCs/10 mL PBS and suspended, and labeled as a final concentration of 1 µM CTV/µL. 1×10⁶ CTV-labeled PBMCs/well were suspended in 200 µL of 10% FBS-containing RPMI1640 culture medium (manufactured by Thermo Fisher Scientific Inc., Cat No: 200-02). A suspension of spores without a heat treatment (spore concentration: 1×10⁷ cells/mL) or a suspension of spores with a heat treatment (spore concentration: 1×10⁷ cells/mL) was added thereto and suspended. Wells without stimulation and wells with stimulation with anti-CD3-CD28 antibody (2 µL) were placed as controls. After the pulse, culturing was performed for 7 days under the conditions of 37°C and 2.5% CO₂. After the culturing, PBMCs were washed, and the surface was stained using an antibody. As antibodies, CD3 BV785 (50-fold dilution, manufactured by BioLegend, Inc.), CD4 BV750 (100-fold dilution, manufactured by BioLegend, Inc.), CD8 BV570 (100-fold dilution, manufactured by BioLegend, Inc.), TCRγδ FITC (100-fold dilution, manufactured by Miltenyi Biotec), TCRab PerCP/Cy5.5 (100-fold dilution, manufactured by BioLegend, Inc.), Vg9 APCvio770 (50-fold dilution, manufactured by Invtrrogen), Vδ1 PE-eFlour610 (50-fold dilution, manufactured by Invtrrogen), and Vδ2 PE (100-fold dilution, manufactured by Miltenyi Biotec) were used. For staining of dead cells, 7-aminoactinomycin D (7-AAD, manufactured by BioLegend, Inc., Cat No: 420404) was also added. After addition, the mixture was incubated on ice for 20 minutes. After incubation, PBMCs were immobilized using 1% paraformaldehyde (manufactured by NACALAI TESQUE, INC., Cat No: 09154-85). After immobilization, the expression level of surface molecules was measured by flow cytometry using Cytek Northern Lights (manufactured by Cytek Japan). Data acquired by flow cytometry was analyzed using FlowJo software v10 (manufactured by Tree Star, Inc.). The results are shown in Figs. 4B and 4C. In Fig. 4B, "None" indicates no stimulation, "Anti-CD3/CD28" indicates an anti-CD3-CD28 antibody, "Spore Heat-inact" indicates a suspension of spores with a heat treatment, and "Spore" indicates a suspension of spores without a heat treatment. In Fig. 4C, "None" indicates no stimulation, "CD3" indicates an anti-CD3-CD28 antibody, "CBM588 Heat-inact" indicates a suspension of spores with a heat treatment, and "CBM588" indicates a suspension of spores without a heat treatment.

As shown in Figs. 4B and 4C, in the group stimulated with a suspension of spores, a significant increase in CD4⁻CD8⁻ T cells was observed compared to the control group regardless of the presence or absence of a heat treatment. It can be seen that the group stimulated with a suspension of spores without a heat treatment proliferates Vγ9Vδ2 T cells known to have an antitumor effect.

### <Test Example 5>

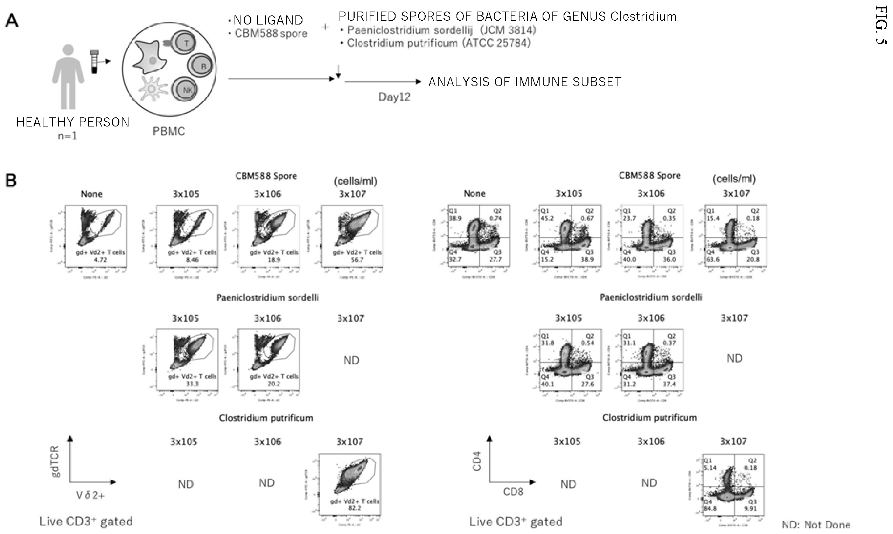

5 mM CTV/20 µL DMSO was diluted 5-fold with PBS by Cell Trace Violet Cell Proliferation Kit (Thermo Fisher Scientific, Invitrogen, C34557) to prepare a 1 mM CTV/µL solution. 10 mM CTV/10 µL was added dropwise to 3×10⁷ PBMCs/10 mL PBS and suspended, and labeled as a final concentration of 1 µM CTV/µL. 3×10⁶ CTV-labeled PBMCs/well were suspended in 200 µL of 10% FBS-containing RPMI1640 culture medium (manufactured by Thermo Fisher Scientific Inc., Cat No: 200-02). A suspension of spores of 588 strains (spore concentration: 3×10⁵ cells/mL, 3×10⁶ cells/mL, or 3×10⁷ cells/mL), a suspension of spores of Clostridium sordellii JCM3814 strains (spore concentration: 3×10⁵ cells/mL or 3×10⁶ cells/mL), or a suspension of spores of Clostridium putrificum ATCC25784 strains (spore concentration: 3×10⁷ cells/mL) was added thereto and suspended. The spores of the added suspension were not subjected to a heat treatment. Wells without stimulation were placed as controls. After the pulse, culturing was performed for 12 days under the conditions of 37°C and 2.5% CO₂. After the culturing, PBMCs were washed, and the surface was stained using an antibody. As antibodies, CD3 BV785 (50-fold dilution, manufactured by BioLegend, Inc.), CD4 BV750 (100-fold dilution, manufactured by BioLegend, Inc.), CD8 BV570 (100-fold dilution, manufactured by BioLegend, Inc.), TCRγδ FITC (100-fold dilution, manufactured by Miltenyi Biotec), TCRab PerCP/Cy5.5 (100-fold dilution, manufactured by BioLegend, Inc.), Vg9APCvio770 (50-fold dilution, manufactured by Invtrrogen), Vδ1 PE-eFlour610 (50-fold dilution, manufactured by Invtrrogen), and Vδ2 PE (100-fold dilution, manufactured by Miltenyi Biotec) were used. For staining of dead cells, 7-aminoactinomycin D (7-AAD, manufactured by BioLegend, Inc., Cat No: 420404) was also added. After addition, the mixture was incubated on ice for 20 minutes. After incubation, PBMCs were immobilized using 1% paraformaldehyde (manufactured by NACALAI TESQUE, INC., Cat No: 09154-85). After immobilization, the expression level of surface molecules was measured by flow cytometry using Cytek Northern Lights (manufactured by Cytek Japan). Data acquired by flow cytometry was analyzed using FlowJo software v10 (manufactured by Tree Star, Inc.). The results are shown in Fig. 5B. In Fig. 5B, "CBM588 Spore" indicates a suspension of spores of 588 strains, "Paeniclostridium sordelli" indicates a suspension of spores of Clostridium sordellii JCM3814 strains, and "Clostridium putrificum" indicates a suspension of spores of Clostridium putrificum ATCC25784 strains.

As shown in Fig. 5, it can be seen that the group stimulated with a suspension of spores of Clostridium sordellii JCM3814 strains and a suspension of spores of Clostridium putrificum ATCC25784 strains proliferates Vγ9Vδ2 T cells known to have an antitumor effect, similar to the group stimulated with a suspension of spores of 588 strains.

## Claims

1. A composition for proliferating and/or activating CD4⁻CD8⁻ T cells, the composition comprising, as an active ingredient, spore-forming bacteria or a cultured material thereof.

2. The composition according to claim 1, wherein the spore-forming bacteria are at least one kind of bacteria selected from a group consisting of bacteria of genus Clostridium and genus Bacillus.

3. The composition according to claim 1, wherein the spore-forming bacteria are at least one kind of bacteria selected from a group consisting of Clostridium butyricum, Clostridium absonum, Clostridium acetobutylicum, Clostridium beijerinckii, Clostridium cadaveris, Clostridium clostridiiforme, Clostridium cochlearium, Clostridium difficile, Clostridium innocuum, Clostridium novyi, Clostridium perfringens, Clostridium putrificum, Clostridium ramosum, Clostridium rectum, Clostridium scatologenes, Clostridium sordellii, Clostridium sporogenes, Clostridium tertium, Clostridium tyrobutyricum, Clostridium aerotolerans, Clostridium aminophilum, Clostridium aminovalericum, Clostridium celerecrescens, Clostridium cellulosi, Clostridium coccoides, Clostridium hiranonis, Clostridium leptum, Clostridium nexile, Clostridium oroticum, Clostridium polysaccharolyticum, Clostridium populeti, Clostridium scindens, Clostridium sphenoides, Clostridium sporosphaeroides, Clostridium symbiosum, Clostridium xylanolyticum, Clostridium hylemonae, Bacillus cereus, Bacillus circulans, Bacillus clausii, Bacillus coagulans, Bacillus licheniformis, Bacillus polyfermenticus, Bacillus pumilus, and Bacillus subtilis.

4. The composition according to claim 1, wherein the spore-forming bacteria are at least one kind of bacteria selected from a group consisting of Clostridium butyricum, Clostridium sordellii, and Clostridium putrificum.

5. The composition according to claim 1, wherein the spore-forming bacteria are in a form of spores.

6. The composition according to claim 1, wherein the CD4⁻CD8⁻ T cells include γδ T cells.

7. The composition according to claim 6, wherein the γδ T cells include Vγ9Vδ2 T cells.

8. The composition according to claim 1, which is used in combination with an antitumor agent.

9. The composition according to claim 1, which is a pharmaceutical composition.

10. The composition according to claim 1, which is a food and beverage composition.

11. A method for proliferating and/or activating CD4⁻CD8⁻ T cells, the method comprising bringing peripheral blood mononuclear cells into contact with spore-forming bacteria or a cultured material thereof.

12. A method for proliferating and/or activating CD4⁻CD8⁻ T cells, the method comprising administering an effective amount of spore-forming bacteria or a cultured material thereof to a subject.

13. Spore-forming bacteria or a cultured material thereof being used for proliferating and/or activating CD4⁻CD8⁻ T cells.
